(19) Europäisches Patentamt
European Patent Office
Office européen des brevets

(11) Veröffentlichungsnummer : **0 373 542 B1**

(12) # EUROPÄISCHE PATENTSCHRIFT

(45) Veröffentlichungstag der Patentschrift : **17.06.92 Patentblatt 92/25**

(21) Anmeldenummer : **89122736.5**

(22) Anmeldetag : **09.12.89**

(51) Int. Cl.[5] : **C07D 233/96,** A61K 31/415, C07D 401/06, C07D 409/06, C07D 405/06, A61K 31/44, A61K 31/38, A61K 31/34

(54) **Neue Imidazolidin-derivate, Verfahren zu ihrer Herstellung sowie Arzneimittel, die diese Verbindingen enthalten.**

Die Akte enthält technische Angaben, die nach dem Eingang der Anmeldung eingereicht wurden und die nicht in dieserPatentschrift enthalten sind.

(30) Priorität : **13.12.88 DE 3841879**

(43) Veröffentlichungstag der Anmeldung : **20.06.90 Patentblatt 90/25**

(45) Bekanntmachung des Hinweises auf die Patenterteilung : **17.06.92 Patentblatt 92/25**

(84) Benannte Vertragsstaaten : **AT BE CH DE ES FR GB GR IT LI LU NL SE**

(56) Entgegenhaltungen :
**DE-B- 1 038 050**
**JOURNAL OF HETEROCYCLIC CHEMISTRY, Band 20, Nr. 1, Februar 1983, Seiten 189-191, Provo, US; SANAA O. ABD ALLAH et al.: "Anovel synthesis of fused imidazo[5,1-c]-1,2,4-triazoles"**

(56) Entgegenhaltungen :
**CHEMICAL ABSTRACTS, Band 83, Nr. 5, 4. August 1975, Seite 494, Spalte 1, Zusammenfassung Nr. 43237p, Columbus, Ohio, US;A.F.A. SHALABY et al.: "Reactions of aromatic amines on 5-arylidene-4-(methylthio)hydantoin and 5-arylazo-2-(methylthio)hydantoin"**

(73) Patentinhaber : **BOEHRINGER MANNHEIM GMBH**
**Sandhofer Strasse 116**
**W-6800 Mannheim 31 (DE)**

(72) Erfinder : **Tsaklakidis, Christos, Dr. phil. nat.**
**Weberstrasse 23**
**W-6940 Weinheim (DE)**
Erfinder : **Bosies, Elmar, Dr. phil.**
**Delpstrasse 11**
**W-6940 Weinheim (DE)**
Erfinder : **Schultz, Michael, Dr. rer. nat.**
**Otto-Beck-Strasse 14**
**W-6800 Mannheim 1 (DE)**
Erfinder : **Haag, Rainer, Dr. rer. nat.**
**Stahlbühlring 1**
**W-6802 Ladenburg (DE)**
Erfinder : **Herrmann, Dieter, Dr. med.**
**An der Neckarspitze 13**
**W-6900 Heidelberg (DE)**
Erfinder : **Pahlke, Wulf, Dr.**
**Arminstrasse 37**
**W-6140 Bensheim (DE)**

EP 0 373 542 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Imidazolidin-Derivate, Verfahren zur ihrer Herstellung sowie Arzneimittel, die diese Verbindungen enthalten.

Allgemein sind aus dem Stand der Technik Immunsuppressiva als solche schon seit längerer Zeit bekannt (Pharmazie unserer Zeit 1, 2-8 (1972) und 12, 20-29 (1983)). Der in diesem Zusammenhang verwendete Ausdruck "Immunsuppression" bezeichnet dabei im allgemeinen die unspezifische Unterdrückung der Immunantwort, z.B. mit Hilfe von Antiseren, ionisierenden Strahlen oder speziellen Therapeutika.

Diese Chemotherapeutika finden Anwendung nach der Transplantation von Geweben oder Organen und bei der Therapie von Autoimmunerkrankungen. Sie hemmen die Proliferation von Lymphozyten durch direkten Eingriff in die DNA- und RNA-Synthese. In diese Klasse von Verbindungen gehören Cyclosporine, Folsäure-Antagonisten, Purin-Analoga, alkylierende Substanzen wie Cyclophosphamid und bestimmte Corticosteroide. Der Nachteil dieser bisher eingesetzten Immunsuppresiva ist jedoch die in erhöhtem Maße zu beobachtende Infektionsanfälligkeit des behandelten Organismus, da das gesamte körpereigene Immunsystem geschwächt und sowohl die humorale als auch die zelluläre Immunantwort unterdrückt werden.

Die immunsuppressiven Eigenschaften von derzeit bekannten Immunsuppressiva, wie z.B. Zytostatika und Corticosteroide, sind dosisabhängig aber unselektiv, d.h. sie wirken auf alle immunkomptenten Zellen. Diese Verbindungen hemmen sowohl die humorale als auch zelluläre Immunantwort auf eine vielzahl von Antigen und wirken unspezifisch auf T- oder B-Lymphozyten.

Es besteht daher sehr großes Interesse an Immunsuppressiva, die spezifisch mit pathologisch verstärken bzw. erhöhten Immunmechanismen interferieren, ohne jedoch die im Körper normal ablaufenden natürlichen Immunreaktionen zu beeinflussen. Solche spezifisch wirksamen immunsuppressiven Substanzen sind bis heute nicht bekannt.

Der Erfindung lag daher die Aufgabe zugrunde, ein solches neues immunsuppressiv wirksames Mittel zur Verfügung zu stellen.

Es wurde nun überraschenderweise gefunden, daß die erfindungsgemäßen Verbindungen diese Aufgabe lösen und als vorteilhafte Immunsuppressiva eingesetzt werden können. Sie supprimieren spezifisch die B-Zell-Proliferation oder B-Zell-Aktivierung. Sie können vorteilhaft bei der Behandlung von allen Krankheiten eingesetzt werden, bei denen eine polyklonale Aktivierung oder Proliferation von B-Zellen von pathophysiologischer, sympthomatischer oder klinischer Relevanz ist.

In diesem Sinne kommt die Behandlung von folgenden Krankheiten in Frage: Autoimmunerkrankungen, wie Rheumatoide Arthritis, Diabetes mellitus Typ I, Psoriasis, Lupus Systemicus Erythematosus; Abstoßungsreaktion nach Gewebe oder Organtransplantationen z.B. von Haut, Knochenmark und Nieren, virale oder retrovirale Infektionen jeglicher Genese, wie z.B. ARC (AIDS related complex) und AIDS sowie deren Vorstadien; sowie B-Zell-Leukämien und Lymphome, z.B. chronisch lymphatische Leukämie, lymphoblastische Lymphome (z.B. Burkitt-Lymphom etc.) oder B-Zell/Plasma-zell-Neoplasien, wie z.B. Plasmazytom (multiples Myelom) etc.. Als Autoimmunerkrankungen werden in der Literatur im allgemeinen solche Erkrankungen bezeichnet, die mit der Bildung von Autoantikörpern in Verbindung stehen. Diese Autoantikörper richten sich gegen körpereigene Antigene und bewirken somit eine Abwehr gegenüber körpereigenen Stoffen. Diese krankhafte Überreaktion des Immunsystems gilt es mit spezifisch wirksamen Immunsuppressiva zu unterdrücken.

Im Sinne der vorliegenden Erfindung soll der Ausdruck "Immunsuppression" im allgemeinen alle Aspekte der natürlicherweise induzierten immunologischen Nicht-Ansprechbarkeit, der artifiziell induzierten Nicht-Ansprechbarkeit und der pathologisch induzierten Toleranz eines Individuums auf Auto- oder Fremdantigene beinhalten.

Gegenstand der Erfindung sind neue Imidazolidin-Derivate der allgmeinen Formel I,

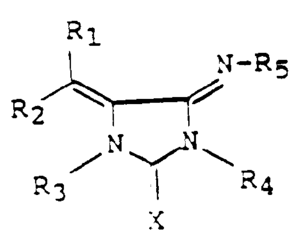

(I)

worin

$R_1$ und $R_2$ gleich oder verschieden sein können und Wasserstoff oder einen $C_1$-$C_5$-Alkylrest, einen $C_3$-$C_5$-Alkenylrest, einen Phenylrest bedeuten oder zusammen mit dem gemeinsamen Kohlenstoffatom einen gesättigten

oder ungesättigten $C_3$-$C_7$-Ring bilden,
$R_3$ und $R_4$ gleich oder verschieden sein können und Wasserstoff, einen geradkettigen oder verzweigten $C_1$-$C_{10}$-Alkylrest, einen geradkettigen oder verzweigten $C_3$-$C_7$-Alkenylrest, einen $C_3$-$C_7$-Cycloalkylrest, einen $C_3$-$C_7$-Cycloalkenylrest, einen Phenyl-, Benzyl- oder Picolyl-, Thenyl- oder Furfuryl-Rest
$R_5$ Wasserstoff oder einen Methyl-, Ethyl- oder Propyl-Rest bedeutet,
und
X Sauerstoff, Schwefel oder N-H bedeutet.

Für den Fall, daß $R_4$ Wasserstoff bedeutet, können die Verbindungen der allgemeinen Formel I auch in der isomeren Form I' vorliegen.

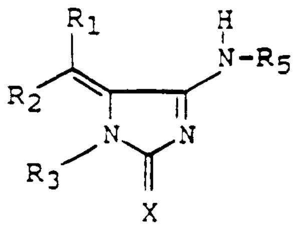

(I')

in der $R_1$, $R_2$, $R_3$ und $R_5$ die bei Formel I angegebene Bedeutung haben.

Der $C_1$-$C_5$-Rest von $R_1$ und $R_2$ bedeutet Methyl, Ethyl, n-Propyl, Isopropyl, Butyl, Isobutyl, Pentyl und Isopentyl, insbesondere Methyl. Der $C_3$-$C_5$-Alkenylrest bedeutet Allyl, Butenyl, Isobutenyl, Pentenyl und Isopentenyl, insbesondere Allyl. Der $C_3$-$C_7$-Ring bedeutet vorzugsweise Cyclopropyl, Cyclopentyl und Cyclohexyl.

Der $C_1$-$C_{10}$-Alkylrest von $R_3$ und $R_4$ bedeutet vorzugsweise Methyl, Ethyl, Propyl, Isopropyl, Butyl, Isobutyl, n-Pentyl, Isopentyl, Neopentyl, n-Hexyl, Isohexyl, n-Octyl, n-Decyl, insbesondere Methyl, Propyl, Isopropyl, Neopentyl und n-Hexyl; der $C_3$-$C_7$-Alkenylrest von $R_3$ und $R_4$ bedeutet vorzugsweise Allyl, Butenyl, Isobutenyl, Pentenyl, Hexenyl, Heptenyl, insbesondere Allyl und Isobutenyl; der $C_3$-$C_7$-Cycloalkylrest bedeutet vorzugsweise Cyclopropyl, Cyclohexyl, Cycloheptyl, insbesondere Cyclopropyl und Cyclohexyl; der $C_3$-$C_7$-Cycloalkenylrest bedeutet vorzugsweise Cyclopentenyl und Cyclohexenyl; der Arylalkyl-Substituent von $R_3$ und $R_4$ bedeutet vorzugsweise den Benzylrest; det Hetarylalkylrest stellt vorzugsweise den Picolyl-, Thenyl-und Furfuryl-Rest dar.

Der niedere Alkylrest von $R_5$ bedeutet vorzugweise Methyl, Ethyl, Propyl.

Die erfindungsgemäßen Verbindungen der allgemeinen Formel I können dargestellt werden, indem man, für den Fall, daß $R_5$ Wasserstoff bedeutet,

a) Verbindungen der allgemeinen Formel II oder III,

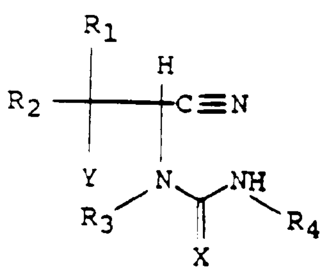

(II)

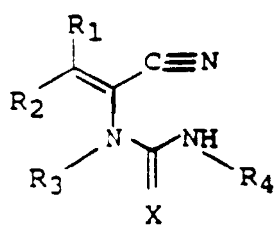

(III)

in denen $R_1$, $R_2$, $R_3$, $R_4$ und X die oben genannten Bedeutungen besitzen und Y in der Formel II eine Abgangsgruppe wie Halogen, Sulfonsäureester oder einen Acyloxy-Rest bedeutet, mit einer Base umsetzt, oder

b) für den Fall, daß X in der Verbindung der Allgemeinen Formel I Schwefel oder N-H bedeutet, die Carbonylgruppe einer Verbindung der allgemeinen Formel IV,

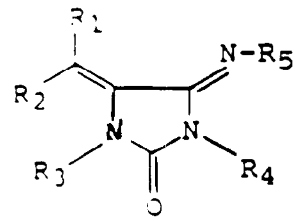

(IV)

in der $R_1$, $R_2$, $R_3$, $R_4$ und $R_5$ die oben gennanten Bedeutungen besitzen, in an sich bekannter Weise in eine Thiocarbonyl-bzw. Iminogruppe überführt,
oder
c) für den Fall, daß in der Verbindung der allgemeinen Formel I der Substituent X Sauerstoff, die Substituenten $R_3$ und $R_4$ die oben genannten Bedeutungen besitzen, jedoch nicht gleichzeitig Wasserstoff oder die Phenylgruppe sein dürfen, eine Verbindung der allgemeinen Formel V oder VI,

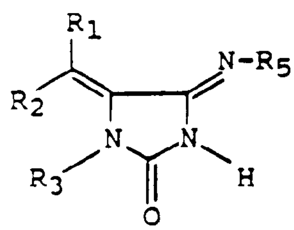

(V)

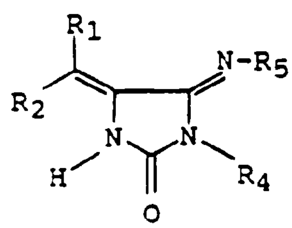

(VI)

in der $R_1$, $R_2$, $R_3$, $R_4$ und $R_5$ dei oben genannten Bedeutungen besitzen, in an sich bekannter Weise mit einer Verbindung der allgemeinen Formel VII,

$Y\text{-}R_5$ (VII)

in der Y die oben genannten Bedeutungen besitzt und $R_6$ die gleiche Bedeutung wie $R_3$ bzw. $R_4$ hat, mit Ausnahme der Bedeutung Wasserstoff und Phenyl, umsetzt,
oder
d) eine Verbindung der allgemeinen Formel VIII,

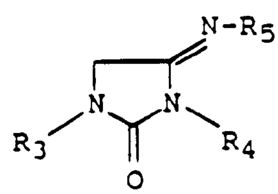

(VIII)

in der $R_3$, $R_4$ und $R_5$ die oben genannten Bedeutungen besitzen, mit einer Verbindung der allgemeinen Formel IX

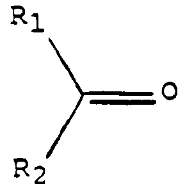

(IX)

in der $R_1$ und $R_2$ die oben genannten Bedeutungen besitzen, mit Hilfe eines Kondensationsmittels zur Reaktion bringt,
oder
e) für den Fall, daß X in der allgemeinen Formel I Sauerstoff bedeutet, eine Verbindung der allgemeinen Formel X,

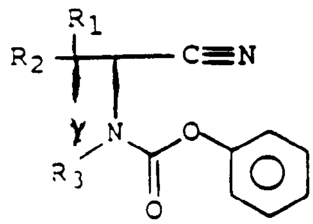

(X)

in der $R_1$, $R_2$, $R_3$ und Y die oben genannten Bedeutungen besitzen, mit einem primären Amin der allgemeinen Formel XI,

$R_4$-$NH_2$ (XI)

in der $R_4$ die oben genannten Bedeutungen besitzt, umsetzt,
oder
f) Verbindungen der allgemeinen Formel I, bei den $R_5$ Wasserstoff bedeutet, gegebenenfalls nachträglich durch Alkylierung in Verbindungen der allgemeinen Formel I mit $R_5$ gleich niederer Alkylrest überführt,
oder
g) für den Fall, daß $R_1$, $R_2$, $R_3$ $R_4$ und $R_5$ in der allgemeinen Formel I Wasserstoff bedeuten, die Verbindung 4-Imino-1,3-diazabicyclo[3.1.0]hexan-2-on einer Hydrolyse unterwirft,
oder
h) für den Fall, daß $R_4$ und $R_5$ Wasserstoff und X Sauerstoff bedeuten, eine Verbindung der allgemeinen Formel XII,

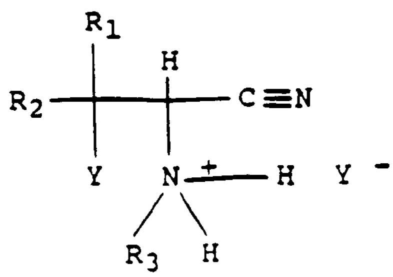

(XII)

in der $R_1$, $R_2$, $R_3$ und Y die oben genannten Bedeutungen besitzen, mit Chlorsulfonylisocyanat umsetzt.

Die Verbindung 4-Imino-1,3-diazabicyclo[3.1.0]hexan-2-on kann nach dem in der DE-OS 25 30 398 beschriebenen Verfahren hergestellt werden.

Als Basen für die Cyclisierung der Verbindungen der allgemeinen Formel II und III zu den erfindungsgemäßen Verbindungen der allgemeinen Formel I kommen in der Regel Alkalilaugen wie wässrige NaOH, wässrige KOH usw., alkoholische NaOH bzw. KOH, Alkalialkoholate wie Natrium- oder Kaliummethylat, Natrium- oder Kaliumethylat, Kalium- tert. Butylat oder Stickstoffbasen wie Triethylamin, 1,5-Diazabicyclo-[4.4.0]non-5-en(DBN) bzw. 1,8-Diazabicyclo-[5.4.0] undec-7-en(DBU) in Frage. Die Reaktion wird in der Regel in Wasser, Alkohol oder in einem inerten Lösungsmittel wie Tetrahydrofuran, Diethylether oder Toluol und bei einer Temperatur zwischen -10°C und 60°C, vorzugsweise bei Raumtemperatur durchgeführt. Setzt man Verbindungen

der allgemeinen Formel II zu den erfindungsgemäßen Verbindungen der allgemeinen Formel I um, so benötigt man mindestens 1.2 Molequivalent Base, während die Cyclisierung der Verbindungen der allgemeinen Formel III zu den Verbindungen der allgmeinen Formel I mit katalytischen Mengen der verwendeten Base durchgeführt wird.

Verbindungen der allgemeinen Formel II bzw. X können hergestellt werden, indem man eine Verbindung der allgemeinen Formel XII, mit einer Verbindung der allgemeinen Formel XIII,

$R_4$-N=C=X (XIII)

in der $R_4$ und X die oben genannten Bedeutungen besitzen, bzw. mit Chlorkohlensäurephenylester in Wasser oder in einem inerten Lösungsmittel wie Toluol, Ether oder Methylenchlorid, oder in einem Zweiphasensystem wie Wasser/Methylenchlorid unter Verwendung einer Base wie NaOH, KOH, $NaHCO_3$, $Na_2CO_3$ oder Triethylamin und bei einer Temperatur zwischen -10°C und 60°C, vorzugsweise bei Raumtemperatur umsetzt.

Verbindungen der allgemeinen Formel III erhält man entweder aus Verbindungen der allgemeinen Formel II, indem man aus letzteren mittels eines Eliminierungsmittels H-Y abspaltet, oder bei der Umsetzung einer Verbindung der allgemeinen Formel X mit einem Amin der allgemeinen Formel IX. Als Eliminierungsmittel eignen sich tertiäre Stickstoffbasen wie Triethylamin, DBN oder DBU. Die Überführung der Carbonylgruppe einer Verbindung der allgemeinen Formel IV in eine Thiocarbonyl- bzw. Iminogruppe erfolgt nach literaturbekannten Methoden wie z.B. durch Umsetzung einer Verbindung der Formel IV mit $P_4S_{10}$ (Liebigs Ann. Chem. 746, 92, (1971)) oder mit Lawesson's-Reagenz (Bul. soc. Chim. Belg., 87, 223, 229, 525 (1978)) bzw. mit Phenylphosphorodiamidat (J. Heterocycl. Chem., 9, 1235 (1972)).

Verbindungen der allgemeinen Formel XII können hergestellt werden, indem man ein Cyanaziridin der allgemeinen Formel XIV,

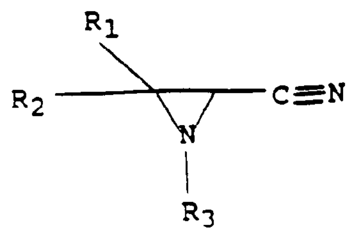

(XIV)

in der $R_1$, $R_2$ und $R_3$ die oben genannten Bedeutungen besitzen, mit zwei Molequivalent einer Säure der allgemeinen Formel XV,

H-Y (XV)

in der Y die oben genannten Bedeutungen besitzt, zur Reaktion bringt.

Verbindungen der allgemeinen Formel XIV können nach dem in der DE 28 33 986 beschriebenen Verfahren hergestellt werden.

Die Umsetzung einer Verbindung der allgemeinen Formel X mit einer Verbindung der allgemeinen Formel XI erfolgt in Wasser, Alkohol oder in einem inerten Lösungsmittel wie Toluol, Methylenchlorid, oder Ether und bei einer Temperatur zwischen -10°C und 60°C.

Die Reaktion einer Verbindung allgemeinen Formel VIII mit einer Verbindung der allgemeinen Formel IX wird in Wasser, Alkohol oder einem inerten Lösungsmittel wie Toluol, oder Ether unter Zusatz eines Kondensationsmittels duchgeführt. Als Kondensationsmittel verwendet man in der Regel Alkalihydroxide wie Kaliumhydroxid, Alkalialkoholate wie Natriumethylat oder Kalim-tert.butylat oder Stickstoffbasen wie Morpholin oder Piperidin (Biochem. J., 29, 2256 (1935)).

Verbindungen der allgemeinen Formel VIII können nach literaturbekannten Methoden hergestellt werden (z.B. A.F.A. Shalaby, H.A. Daboun, Z. Naturforsch. 306, 124 (1975)).

Die Umsetzung von Verbindungen der allgemeinen Formel XII mit Chlorsulfonylisocyanat erfolgt nach literaturbekannten Verfahren (A.V. Narender Reddy, Synth.Commun., 18 (5) , 525 (1988)).

Zur Herstellung pharmazeutischer Mittel werden die erfindungsgemäßen Verbindungen in an sich bekannter Weise mit geeigneten pharmazeutischen Trägersubstanzen gemischt, gegebenenfalls granuliert und beispielsweise zu Tabletten oder Drageekernen verpreßt. Ebenso ist eine Abfüllung der Mischung in Steckkapseln möglich. Unter Zugabe entsprechender Hilfsstoffe kann auch eine Lösung oder Suspension in Wasser, Öl (z.B. Olivenöl) oder hochmolekularen Polymeren (z.B. Polyethylenglykol) hergestellt und zu Injektionslösungen, Weichgelantiekapseln, Saft oder Tropfen verabreicht werden.

Als feste Trägerstoffe können z.B. Stärken bzw. Stärkederivate, Zucker, Zuckeralkohole, Cellulosen bzw. Cellulosederivate, Tenside, Talkum, hochdisperse Kieselsäuren, hochmolekulare Fettsäuren oder deren Salze, Gelatine, Agar-Agar, Kalziumphosphat, tierische oder pflanzliche Fette oder Wachse und feste hochmolekulare

Polymere (wie Polyethylenglykole oder Polyvinylpyrrolidone) Verwendung finden. Für orale Applikationen geeignete Zubereitungen können gewünschtenfalls Geschmacks- und Süßstoffe enthalten.

Als besonders geeignete Arzneimittelzubereitung hat sich eine Filmtablette mit 100 mg Wirkstoff erwiesen, die wie folgt zusammengesetzt ist:

| | Gewicht/Stück/mg |
|---|---|
| Wirkstoff | 100.000 |
| Lactose x 1 $H_2O$ | 63.000 |
| Poly (O-carboxymethyl)stärke, Na-Salz | 7.000 |
| Poly (1-vinyl-2-pyrrolidon) 25.000 | 4.000 |
| Poly (O-carboxymethylstärke, Na-Salz | 3.000 |
| Mikrokristalline Cellulose | 20.000 |
| Siliciumdioxid, hochdispers | 1.500 |
| Magnesiumstearat | 1.500 |
| Kerngewicht: | 200.000 |

Die Filmtabletten werden dann durch Filmdragierung der erhaltenen Wirkstoffkerne in üblicher Weise hergestellt.

Filmtabletten mit z.B. 10 mg, 200 mg oder 500 mg Wirkstoff werden in entsprechender Weise hergestellt.

Die Dosierung des Wirkstoffes hängt vom Alter und Geschlecht des Individuums sowie von der Art der zu behandelnden Indikation ab.

Grundsätzlich kann davon ausgegangen werden, daß 0.1 - 100 mg pro kg Körpergewicht täglich oral, intravenös subkutan oder intramuskulär appliziert werden können. Bevorzugt sind jedoch Mengen von 5- 50 mg/kg Körpergewicht, insbesondere 5- 20 mg/kg Körpergewicht. Die Wirkstoffmengen können 1 bis 3 mal täglich verabreicht werden.

Neben den nachfolgenden aufgeführten Beispielen sind insbesondere die folgenden Verbindungen im Sinne der Anmeldung bevorzugt:

1) 4-Imino-5-methylen-imidazolidin-2-thion
2) 2,4-Diimino-5-methylen-imidazolidin
3) 4-Imino-5-n-hexyliden-imidazolidin-2-on
4) 1-Methyl-4-imino-5-methylen-imidazolidin-2-on
5) 1-Isopropyl-4-imino-5-methylen-imidazolidin-2-on
6) 1-n-Hexyl-4-imino-5-methylen-imidazolidin-2-on
7) 1-Allyl-4-imino-5-methylen-imidazolidin-2-on
8) 1-Cyclopropyl-4-imino-5-methylen-imidazolidin-2-on
9) 1-(3-Pyridylmethyl)-4-imino-5-methylen-imidazolidin-2-on
10) 1-Phenyl-4-imino-5-methylen-imidazolidin-2-on
11) 3-Methyl-4-imino-5-methylen-imidazolidin-2-on Fp. 230°C (Zers.)
12) 3-n-Propyl-4-imino-5-methylen-imidazolidin-2-on Fp. 130°C (Zers.)
13) 3-(2,2-Dimethyl-propyl)-4-imino-5-methylen-imidazolidin-2-on
14) 3-Cyclohexyl-4-imino-5-methylen-imidazolidin-2-on Fp. 163-164°C
15) 3-Isobutenyl-4-imino-5-methylen-imidazolidin-2-on
16) 3-(4-Pyridylmethyl)-4-imino-5-methylen-imidazolidin-2-on Fp. 194-196°C
17) 3-Phenyl-4-imino-5-methylen-imidazolidin-2-on Fp. 208-210°C
18) 1,3-Dimethyl-4-imino-5-methylen-imidazolidin-2-on
19) 4-Imino-5-cyclohexyliden-imidazolidin-2-on
20) 4-Imino-5-benzyliden-imidazolidin-2-on
21) 1,3-Dimethyl-4-methylimino-imidazolidin-2-on
22) 1,3-Di-n-propyl-4-propylimino-imidazolidin-2-on

**Beispiel 1**

**4-Imino-5-methylen-imidazolidin-2-on**

Zu der Lösung von 2.79 g (19.8 mmol) 2-Amino-3-chlorpropionitril-Hydrochlorid (Fp. 160°C) in 20 ml 2 N Natriumcarbonatlösung tropft man bei Raumtemperatur und unter gutem Rühren gleichzeitig die Lösung von 3.2 g (20 mmol) Chlorameisensäurephenylester in 20 ml Diethylether und 20 ml 2 N Natriumcarbonatlösung zu. Danach läßt man die Reaktionsmischung noch 2 Stunden bei Raumtemperatur rühren, trennt dann die Etherphase ab, wäscht sie einmal mit Wasser und trocknet sie über Natriumsulfat. Nach Abziehen des Lösungsmittels unter vermindertem Druck wird der Rückstand aus Toluol umkristallisiert. Man erhält 3.82 g (85 %) 2-(Phenoxycarbonylamino)-3-chlorpropionitril vom Fp. 103 -105°C.

Die Suspension von 3,25 g (14.5 mmol) 2-(Phenoxycarbonyl-amono)-3-chlorpropionitril in 30 ml 2 N Ammoniak läßt man 1 Stunde bei Raumtemperatur rühren, saugt dann den ausgefallenen Niederschlag ab und waescht mit wenig kaltem Waser und Diethylether nach. Man erhält 1.24 g (66 %) der Titelverbindung, die 1 mol Kristallwasser enthält. Fp.: >300°C.

**Beispiel 2**

Ausgehend von 2-Amino-3-chlorbutyronitril-Hydrochlorid (Fp. 181°C) wurde analog hergestellt:

**4-Imino-5-ethyliden-imidazolidin-2-on.** Ausbeute: 69 %, Fp.: 252°C

**Beispiel 3**

**4-Imino-5-isopropyliden-imidazolidin-2-on**

Die Lösung von 6 g (62.5 mmol) 2-Cyano-3,3-dimethyl-aziridin in 30 ml 6 N Salzsäure wird 45 min bei 50°C gerührt. Anschließend wird die Lösung zur Trockne eingedampft und der Rückstand aus Isopropanol umkristallisiert. Man erhält 2.2 g (21 %) 2-Amino-3-chlor-3-Methylbutyronitril-Hydrochlorid. Fp.: 182-184°C.

Zu der lösung von 2.2 g (13 mmol) 2-Amino-3-chlor-3-methylbutyronitril-Hydrochlorid in 15 ml 2 N Natriumcarbonatlösung tropft man bei Raumtemperatur und unter gutem Rühren gleichzeitig die Lösung von 2.24 g (14.3 mmol) Chlorameisensäurephenylester in 15 ml Diethylether und 15 ml 2 N Natriumcarbonatlösung zu. Danach läßt man die Reaktionsmischung noch 2 Stunden bei Raumtemperatur rühren, trennt die Etherphase ab, wäscht sie einmal mit Wasser und trocknet sie über Natriumsulfat. Nach Abziehen des Lösungsmittels unter vermindertem Druck wird der Rückstand aus Toluol umkristallisiert. Man erhält 3,2 g (98 %) 2-(N-Phenoxycarbonyl)amino-3-chlor-3-methyl-butyronitril vom Fp. 98 -100°C.

Die Suspension von 2 g (7.9 mmol) 2-(N-Phenoxycarbonyl)amino-3-chlor-3-methyl-butyronitril in 20 ml 2 N Ammoniak läßt man 4 Stunden bei Raumtemperatur rühren. Dann wird der Niederschlag abgesaugt und mit wenig kaltem Wasser und Diethylether gewaschen. Man erhält 0.7 g (64 %) 2-(N-Aminocarbonyl)amino-3-methyl-crotonsäurenitril. Fp.: 204 - 206°C.

Zu der Suspension von 0.42 g (3 mmol) 2-(N-Aminocarbonyl)amino-3-methyl-crotonsäurenitril in 6 ml absolutem Ethanol tropft man unter Rühren innerhalb 5 min. eine Lösung von 10 mg Natrium in 2 ml absolutem Ethanol zu. Anschließend läßt man die Reaktionsmischung noch 2.5 Stunden bei Raumtemperatur rühren, saugt dann den Niederschlag ab und wäscht ihn mit Ethanol und Diethylether. Man erhält 0.32 g (76.6 % der Titelverbindung vom Fp. 258°C (Zersetzung).

**Beispiel 4**

**3-Benzyl-4-imino-5-methylen-imidazolidin-2-on**

Die Lösung von 1,35 ml (11 mMol) Benzylisocyanat in 10 ml Methylenchlorid tropft man unter kräftigem Rühren bei Raumtemperatur zu einer Lösung von 1,41 g (10 mMol) 2-Amino-3-chlor-propionitril-Hydrochlorid in 11 ml 1 N Natriumcarbonatlösung. Anschließend läßt man die Reaktionsmischung noch 2 h bei Raumtemperatur rühren, trennt dann die organische Phase ab, wäscht sie mit Wasser und trocknet sie über Natriumsulfat. Nach Abziehen des Lösungsmittels wird der Rückstand aus Ether umkristallisiert. Man erhält 1,75 g (73,8 %) 2-(Benzylaminocarbonyl)amino-3-chlor-propionitril. Fp.: 110-112°C.

Die Lösung von 4,75 g (20 mMol) 2-(Benzylaminocarbonyl) amono-3-chlor-propionitril und 6 ml Triethylamin in 50 ml trock. Tetrahydrofuran wird 5 h am Rückfluß erhitzt. Anschließend wird der ausgefallene Niederschlag abfiltriert, das Filtrat zu Trockne eingedampft und der Rückstand an Kieselgel chromatographiert (Laufmittel: Essigsäureethylester). Man erhält 2,5 g (62,2 %) der Titelverbindung vom Fp.: 139-141°C.

**Beispiel 5**

**1-Benzyl-4-imino-5-methylen-imidazolidin-2-on**

Die Lösung von 6 g (37,8 mMol) 1-Benzyl-2-cyano-aziridin in 30 ml 6 N Salzsäure wurde 1 h bei Raumtemperatur gerührt. Anschließend wurde der ausgefallene Niederschlag abgesaugt und nacheinander mit wenig kaltem Wasser, Isopropanol und Diethylether gewaschen und im Exsikkator getrocknet. Man erhielt 5,4 g (62 %) 2-Benzylamino-3-chlorpropionitril-Hydrochlorid vom Fp.: 161-162°C.

Zu der eisgekühlten Lösung von 4 g (17,3 mMol) 2-Benzylamino-3-chlor-propionitril-Hydrochlorid in 40 ml trock. Methylenchlorid und 2,4 ml Triethylamin tropft man die Lösung von 1,64 ml (18,9 mMol) Chlorsulfonylisocyanat in 20 ml trock. Methylenchlorid. Anschließend läßt man die Reaktionslösung noch 2 h bei 0°C rühren, erwärmt sie dann auf Raumtemperatur und versetzt sie mit 300 ml 5 %ige Natriumhydrogenkarbonat-Lösung. Die organische Phase wird dann abgetrennt, über Natriumsulfat getrocknet und zu Trockne eingedampft. Anschließend wird der Rückstand an Kieselgel chromatographiert (Laufmittel: Methanol/Methylenchlorid = 1/10). Man erhält 0,8 g (24 %) der Titelverbindung vom Fp.: 225°C.

## Patentansprüche

1. Verbindungen der Formel I

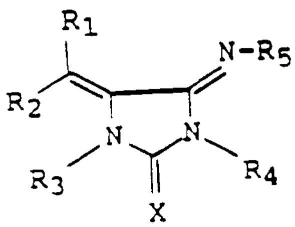

(I)

in der

$R_1$ und $R_2$ gleich oder verschieden sein können und Wasserstoff oder einen $C_1$-$C_5$-Alkylrest, einen $C_3$-$C_5$-Alkenylrest, einen Phenylrest bedeuten oder zusammen mit dem gemeinsamen Kohlenstoffatom einen gesättigten oder ungesättigten $C_3$-$C_7$-Ring bilden,
$R_3$ und $R_4$ gleich oder verschieden sein können und Wasserstoff, einen geradkettigen oder verzweigten $C_1$-$C_{10}$-Alkylrest, einen geradkettigen oder verzweigten $C_3$-$C_7$-Alkenylrest, einen $C_3$-$C_7$-Cycloalkylrest, einen $C_3$-$C_7$-Cycloalkenylrest, einen Phenyl-, Benzyl- oder Picolyl-, Thenyl- oder Furfuryl-Rest bedeuten,
$R_5$ Wasserstoff oder einen Methyl-, Ethyl,- oder Propyl-Rest bedeutet,
und
X Sauerstoff, Schwefel oder N-H bedeuten.

2. Verbindung der Formel I'

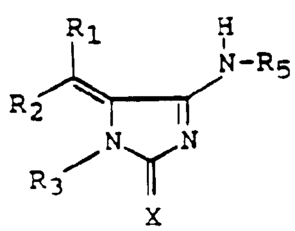

(I')

in der

$R_1$ und $R_2$ gleich oder verschieden sein können und Wasse rstoff oder einen $C_1$-$C_5$-Alkylrest, einen $C_3$-$C_5$-Alkenylrest, einen Phenylrest bedeuten oder zusammen mit dem gemeinsamen Kohlenstoffatom einen gesättigten oder ungesättigten $C_3$-$C_7$-Ring bilden,

$R_3$ Wasserstoff, einen geradkettigen oder verzweigten $C_1$-$C_{10}$-Alkylrest, einen geradkettigen oder verzweigten $C_3$-$C_7$-Alkenylrest, einen $C_3$-$C_7$-Cycloalkylrest, einen $C_3$-$C_7$-Cycloalkenylrest, einen Phenyl-, Benzyl- oder Picolyl-, Thenyl- oder Furfuryl-Rest bedeuten,
$R_5$ Wasserstoff oder einen Methyl-, Ethyl- oder Propyl-Rest bedeutet,
und
X Sauerstoff, Schwefel oder N-H bedeuten.

3. Verfahren zur Herstellung von Verbindungen der Formel

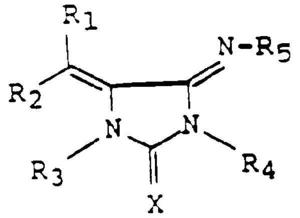

(I)

in der
$R_1$ und $R_2$ gleich oder verschieden sein können und Wasse rstoff oder einen $C_1$-$C_5$-Alkylrest, einen $C_3$-$C_5$-Alkenylrest, einen Phenylrest bedeuten oder zusammen mit dem gemeinsamen Kohlenstoffatom einen gesättigten oder ungesättigten $C_3$-$C_7$-Ring bilden,
$R_3$ und $R_4$ gleich oder verschieden sein können und Wasserstoff, einen geradkettigen oder verzweigten $C_1$-$C_{10}$-Alkylrest, einen geradkettigen oder verzweigten $C_3$-$C_7$-Alkenylrest, einen $C_3$-$C_7$-Cycloalkylrest, einen $C_3$-$C_7$-Cycloalkenylrest, einen Phenyl-, Benzyl-, Picolyl-, Thenyl- oder Furfuryl-Rest bedeuten,
$R_5$ Wasserstoff oder einen Methyl-, Ethyl- oder Propyl-Rest bedeutet,
und
X Sauerstoff, Schwefel oder N-H bedeutet.
dadurch gekennzeichnet, daß man

a) Verbindungen der allgemeinen Formel II oder III,

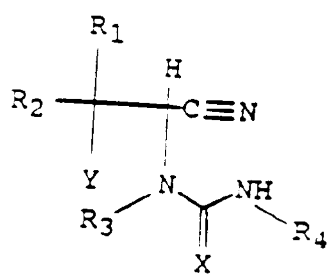

(II)

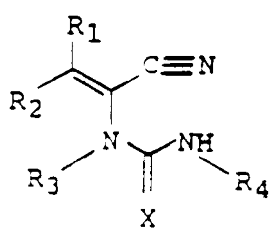

(III)

in denen $R_1$, $R_2$, $R_3$, $R_4$ und X die oben genannten Bedeutungen besitzen und Y in der Formel II eine Abgangsgruppe wie Halogen, Sulfonsäureester oder einen Acyloxy-Rest bedeutet, mit einer Base umsetzt, oder

b) für den Fall, daß X in der Verbindung der allgemeinen Formel I Schwefel oder N-H bedeutet, die Carbonylgruppe einer Verbindung der Allgemeinen Formel IV,

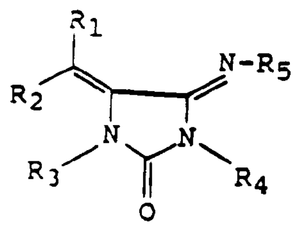

(IV)

in der $R_1$, $R_2$, $R_3$, $R_4$ und $R_5$ die oben genannten Bedeutungen besitzen, in an sich bekannter Weise in eine Thiocarbonyl-bzw. Iminogruppe überführt,

oder

c) für den Fall, daß in der Verbindung der allgemeinen Formel I der Substituent X Sauerstoff, die Substituenten $R_3$ und $R_4$ die oben genannten Bedeutungen besitzen, jedoch nicht gleichzeitig Wasserstoff oder die Phenylgruppe sein dürfen, eine Verbindung der allgemeinen Formel V oder VI,

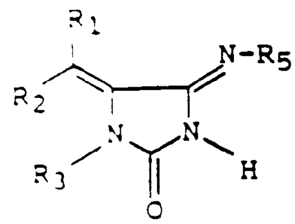

(V)

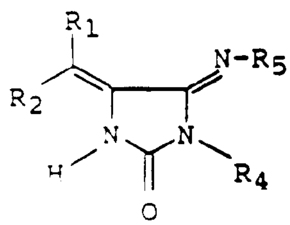

(VI)

in der $R_1$, $R_2$, $R_3$, $R_4$ und $R_5$ die oben genannten Bedeutungen besitzen, in an sich bekannter Weise mit einer Verbindung der allgemeinen Formel VII,

$$Y\text{-}R_6 \quad \text{(VII)}$$

in der Y die oben genannten Bedeutungen besitzt und $R_6$ die gleiche Bedeutung wie $R_3$ bzw. $R_4$ hat, mit Ausnahme der Bedeutung Wasserstoff und Phenyl, umsetzt,

oder

d) eine Verbindung der allgemeinen Formel VIII,

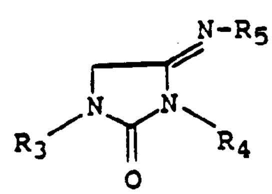

(VIII)

in der $R_3$, $R_4$ und $R_5$ die oben genannten Bedeutungen besitzen, mit einer Verbindung der allgemeinen Formel IX

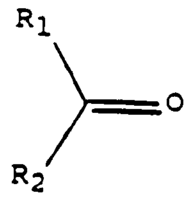

(IX)

in der $R_1$ und $R_2$ die oben genannten Bedeutungen besitzen, mit Hilfe eines Kondensationsmittels zur Reaktion bringt,
oder
e) für den Fall, daß X in der allgemeinen Formel I Sauerstoff bedeutet, eine Verbindung der allgemeinen Formel X,

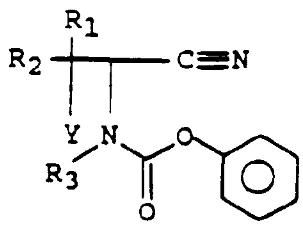

(X)

in der $R_1$, $R_2$, $R_3$ und Y die oben genannten Bedeutungen besitzen, mit einem primären Amin der allgemeinen Formel XI,

$R_4$-$NH_2$ (XI)

in der $R_4$ die oben genannten Bedeutungen besitzt, umsetzt,
oder
f) Verbindungen der allgemeinen Formel I, bei den $R_5$ Wasserstoff bedeutet, gegebenenfalls nachträglich durch Alkylierung in Verbindungen der allgemeinen Formel I mit $R_5$ gleich niederer Alkylrest überführt,
oder
g) für den Fall, daß $R_1$, $R_2$, $R_3$, $R_4$ und $R_5$ in der allgemeinen Formel I Waserstoff bedeuten, die Verbindung 4-Imino-1,3-diazabicyclo[3.1.0]hexan-2-on einer Hydrolyse unterwirft,
oder
h) für den Fall, daß $R_4$ und $R_5$ Wasserstoff und X Sauerstoff bedeuten, eine Verbindung der allgemeinen Formel XII,

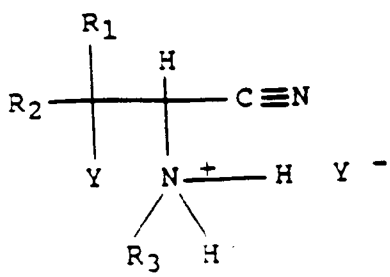

(XII)

in der $R_1$, $R_2$, $R_3$ und Y die oben genannten Bedeutungen besitzen, mit Chlorsulfonylisocyanat umsetzt, und anschließend gewünschtenfalls eine Verbindung der Formel I in eine andere Verbindung der Formel I über führt.

4. Verfahren zur Herstellung von Verbindungen der Formel I′ gemäß Anspruch 3

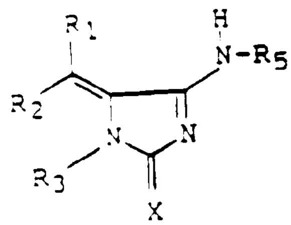

(I')

in der
$R_1$ und $R_2$ gleich oder verschieden sein können und Wasse rstoff oder einen $C_1$-$C_5$-Alkylrest, einen $C_3$-$C_5$-Alkenylrest, einen Phenylrest bedeuten oder zusammen mit dem gemeinsamen Kohlenstoffatom einen gesättigten oder ungesättigten $C_3$-$C_7$-Ring bilden,
$R_3$ Wasserstoff, einen geradkettigen oder verzweigten $C_1$-$C_{10}$-Alkylrest, einen geradkettigen oder verzweigten $C_3$-$C_7$-Alkenylrest, einen $C_3$-$C_7$-Cycloalkylrest, einen $C_3$-$C_7$-Cycloalkenylrest, einen Phenyl-, Benzyl-, Picolyl-, Thenyl- oder Furfuryl-Rest bedeuten,
$R_5$ Wasserstoff oder einen Methyl-, Ethyl- oder Propyl-Rest bedeutet,
und
X Sauerstoff, Schwefel oder N-H bedeuten.

5. Arzneimittel, enthaltend mindest eine der Verbindungen gemäß Ansprüche 1 oder 2 neben üblichen Träger- und Hilfsstoffen.

6. Verwendung von Verbindungen gemäß Ansprüche 1 oder 2 als Immunsuppressiva.

7. Verwendung von Verbindungen gemäß Ansprüche 1 oder 2 zur Herstellung von Arzneimitteln mit immunsuppressiver Wirkung.

## Claims

1. Compounds of the formula I

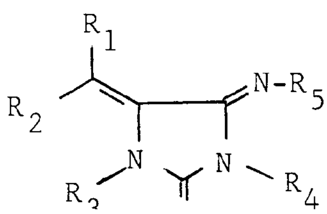

(I)

in which
$R_1$ and $R_2$ can be the same or different and signify hydrogen or a $C_1$-$C_5$-alkyl radical, a $C_3$-$C_5$-alkenyl radical, a phenyl radical or, together with the common carbon atom, form a saturated or unsaturated $C_3$-$C_7$ ring,
$R_3$ and $R_4$ can be the same or different and signify hydrogen, a straight-chained or branched $C_1$-$C_{10}$-alkyl radical, a straight-chained or branched $C_3$-$C_7$-alkenyl radical, a $C_3$-$C_7$-cycloalkyl radical, a $C_3$-$C_7$-cycloalkenyl radical, a phenyl, benzyl or picolyl, thenyl or furfuryl radical,
$R_5$ signifies hydrogen or a methyl, ethyl or propyl radical
and
X signifies oxygen, sulphur or N-H.

2. Compounds of the formula I'

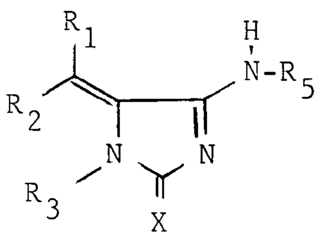

(I')

in which
$R_1$ and $R_2$ can be the same or different and signify hydrogen or a $C_1$-$C_5$-alkyl radical, a $C_3$-$C_5$-alkenyl radical, a phenyl radical or, together with the common carbon atom, form a saturated or unsaturated $C_3$-$C_7$ ring,
$R_3$ signifies hydrogen, a straight-chained or branched $C_1$-$C_{10}$-alkyl radical, a straight-chained or branched $C_3$-$C_7$-alkenyl radical, a $C_3$-$C_7$-cycloalkyl. radical, a $C_3$-$C_7$-cycloalkenyl radical, a phenyl, benzyl or picolyl, thenyl or furfuryl radical,
$R_5$ signifies hydrogen or a methyl, ethyl or propyl radical
and
X signifies oxygen, sulphur or N-H.

3. Process for the preparation of compounds of the formula

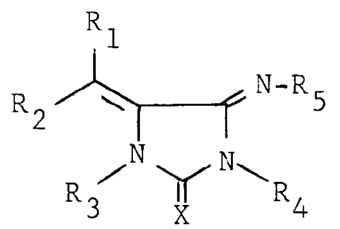

(I)

in which
$R_1$ and $R_2$ can be the same or different and signify hydrogen or a $C_1$-$C_5$-alkyl radical, a $C_3$-$C_5$-alkenyl radical, a phenyl radical or, together with the common carbon atom, form a saturated or unsaturated $C_3$-$C_7$ ring,
$R_3$ and $R_4$ can be the same or different and signify hydrogen, a straight-chained or branched $C_1$-$C_{10}$-alkyl radical, a straight-chained or branched $C_3$-$C_7$-alkenyl radical, a $C_3$-$C_7$-cycloalkyl radical, a $C_3$-$C_7$-cycloalkenyl radical, a phenyl, benzyl, picolyl, thenyl or furfuryl radical,
$R_5$ signifies hydrogen or a methyl, ethyl or propyl radical
and
X signifies oxygen, sulphur or N-H,
characterised in that one

a) reacts compounds of the general formula II or III

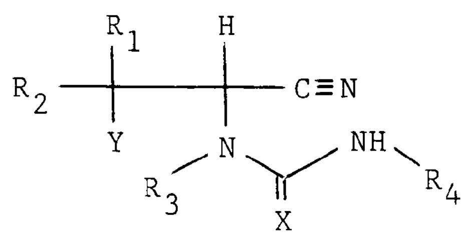

(II)

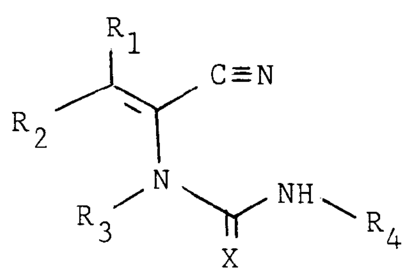

(III)

in which $R_1$, $R_2$, $R_3$, $R_4$ and X possess the above-given meanings and Y in formula II signifies a group which can be removed, such as halogen, sulphonic acid ester or an acyloxy radical, with a base,

or

b) for the case that X in the compound of the general formula I signifies sulphur or NH, converts the carbonyl group of a compound of the general formula IV

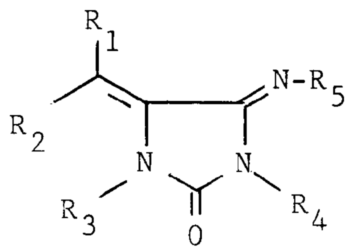

(IV)

in which $R_1$, $R_2$, $R_3$, $R_4$ and $R_5$ possess the above-given meanings, in per se known manner into a thiocarbonyl or imino group,

or

c) for the case that in the compound of the general formula I, the substituent X is oxygen, the substituents $R_3$ and $R_4$ possess the above-given meanings but are not simultaneously hydrogen or the phenyl group, reacts a compound of the general formula V or VI

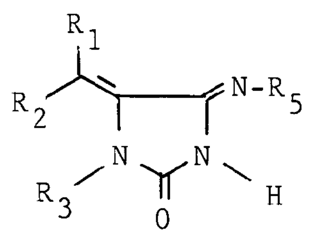

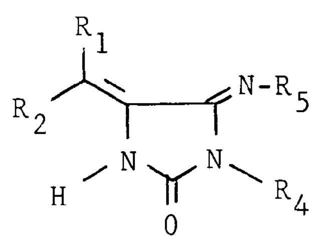

in which $R_1$, $R_2$, $R_3$, $R_4$ and $R_5$ possess the above-given meanings, in per se known manner with a compound of the general formula VII

$$Y - R_6 \qquad (VII)$$

in which Y possesses the above-given meanings and $R_5$ has the same meaning as $R_3$ or $R_4$, with the exception of the meaning hydrogen and phenyl,

or

d) brings a compound of the general formula VIII

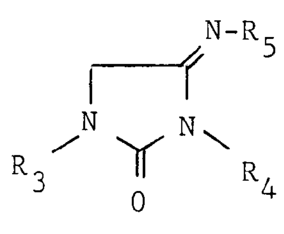

(VIII)

in which $R_3$, $R_4$ and $R_5$ possess the above-given meanings, to reaction with a compound of the general

formula IX

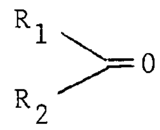

(IX)

in which $R_1$ and $R_2$ possess the above-given meanings, with the help of a condensation agent,
or
e) for the case that X in the general formula I signifies oxygen, reacts a compound of the general formula X

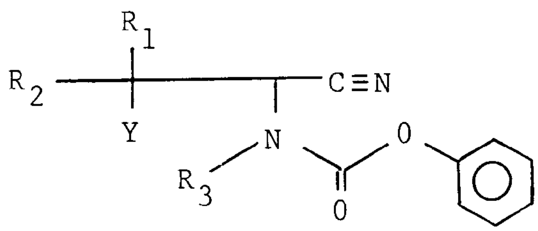

(X)

in which $R_1$, $R_2$, $R_3$ and Y possess the above-given meanings, with a primary amine of the general formula XI

$$R_4 - NH_2 \qquad (XI)$$

in which $R_4$ possesses the above-given meanings,
or
f) possibly subsequently converts compounds of the general formula I, in which $R_5$ signifies hydrogen, by alkylation into compounds of the general formula I with $R_5$ equal to a lower alkyl radical,
or
g) for the case that $R_1$, $R_2$, $R_3$, $R_4$ and $R_5$ in the general formula I signify hydrogen, subjects the compound 4-imino-1,3-diazabicyclo[3.1.0]hexan-2-one to a hydrolysis,
or
h) for the case that $R_4$ and $R_5$ signify hydrogen and X oxygen, reacts a compound of the general formula XII

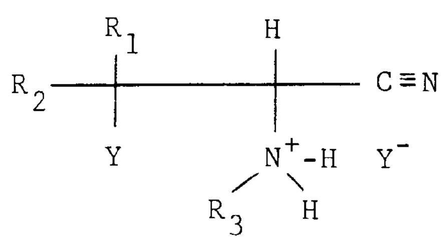

in which $R_1$, $R_2$, $R_3$ and X possess the above-given meanings, with chlorosulphonyl isocyanate,
and
subsequently, if desired, converts a compound of the formula I into another compound of the formula I.

4. Process for the preparation of compounds of the formula I′ according to claim 3

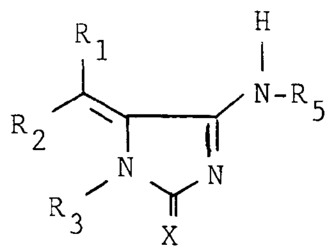

(I')

in which

$R_1$ and $R_2$ can be the same or different and signify hydrogen or a $C_1$-$C_5$-alkyl radical, a $C_3$-$C_5$-alkenyl radical, a phenyl radical or, together with the common carbon atom, form a saturated or unsaturated $C_3$-$C_7$ ring,

$R_3$ signifies hydrogen, a straight-chained or branched $C_1$-$C_{10}$-alkyl radical, a straight-chained or branched $C_3$-$C_7$-alkenyl radical, a $C_3$-$C_7$-cycloalkyl radical, a $C_3$-$C_7$-cycloalkenyl radical, a phenyl, benzyl, picolyl, thenyl or furfuryl radical,

$R_5$ signifies hydrogen or a methyl, ethyl or propyl radical

and

X signifies oxygen, sulphur or N-H.

5. Medicaments containing at least one of the compounds according to claim 1 or 2, besides usual carrier and adjuvant materials.

6. Use of compounds according to claims 1 or 2 as immunosuppressives.

7. Use of compounds according to claims 1 or 2 for the preparation of medicaments with immunosuppressive action.

**Revendications**

1. Composés de formule I

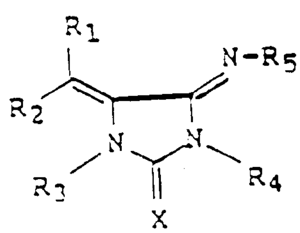

(I)

dans laquelle

$R_1$ et $R_2$ peuvent être identiques ou différents et représentent un atome d'hydrogène ou un groupe alkyle en $C_1$-$C_5$, un groupe alcényle en $C_3$-$C_5$, un groupe phényle ou forment, conjointement avec l'atome de carbone commun, un cycle en $C_3$-$C_7$ saturé ou insaturé,

$R_3$ et $R_4$ peuvent être identiques ou différents et représentent un atome d'hydrogène, un groupe alkyle en $C_1$-$C_{10}$ à chaîne droite ou ramifiée, un groupe alcényle en $C_3$-$C_7$ à chaîne droite ou ramifiée, un groupe cycloalkyle en $C_3$-$C_7$, un groupe cycloalcényle en $C_3$-$C_7$, un groupe phényle, benzyle ou picolyle, thényle ou furfuryle,

$R_5$ représente un atome d'hydrogène ou un groupe méthyle, éthyle ou propyle,

et

X représente un atome d'oxygène, un atome de soufre ou un groupe N-H.

2. Composé de formule I'

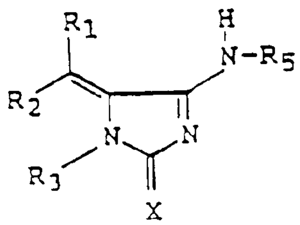

(I')

dans laquelle $R_1$ et $R_2$ peuvent être identiques ou différents et représentent un atome d'hydrogène ou un groupe alkyle en $C_1$-$C_5$, un groupe alcényle en $C_3$-$C_5$, un groupe phényle ou forment, conjointement avec l'atome de carbone commun, un cycle en $C_3$-$C_7$ saturé ou insaturé,
$R_3$ représente un atome d'hydrogène, un groupe alkyle en $C_1$-$C_{10}$ à chaîne droite ou ramifiée, un groupe alcényle en $C_3$-$C_7$ à chaîne droite ou ramifiée, un groupe cycloalkyle en $C_3$-$C_7$, un groupe cycloalcényle en $C_3$-$C_7$, un groupe phényle, benzyle ou picolyle, thényle ou furfuryle,
$R_5$ représente un atome d'hydrogène ou un groupe méthyle, éthyle ou propyle,
et
X représente un atome d'oxygène, un atome de soufre ou un groupe N-H.

3. Procédé pour la préparation de composés de formule

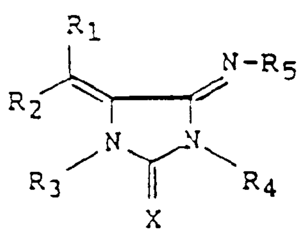

(I)

dans laquelle
$R_1$ et $R_2$ peuvent être identiques ou différents et représentent un atome d'hydrogène ou un groupe alkyle en $C_1$-$C_5$, un groupe alcényle en $C_3$-$C_5$, un groupe phényle ou forment, conjointement avec l'atome de carbone commun, un cycle en $C_3$-$C_7$ saturé ou insaturé,
$R_3$ et $R_4$ peuvent être identiques ou différents et représentent un atome d'hydrogène, un groupe alkyle en $C_1$-$C_{10}$ à chaîne droite ou ramifiée, un groupe alcényle en $C_3$-$C_7$ à chaîne droite ou ramifiée, un groupe cycloalkyle en $C_3$-$C_7$, un groupe cycloalcényle en $C_3$-$C_7$, un groupe phényle, benzyle, picolyle, thényle ou furfuryle,
$R_5$ représente un atome d'hydrogène ou un groupe méthyle, éthyle ou propyle,
et
X représente un atome d'oxygène, un atome de soufre ou un groupe N-H,
caractérisé en ce que

a) on fait réagir des composés de formule générale II ou III,

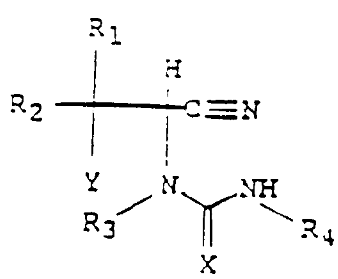

(II)

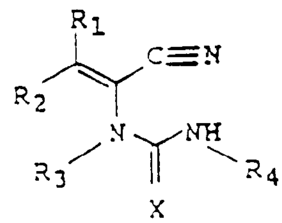

(III)

dans laquelle $R_1$, $R_2$, $R_3$, $R_4$ et X ont les significations mentionnées ci-dessus et Y dans la formule II représente un groupe partant tel qu'un atome d'halogène, un ester d'acide sulfonique ou un groupe acyloxy, avec une base

ou bien

b) dans le cas où X dans le composé de formule générale I représente un atome de soufre ou un groupe N-H, on transforme le groupe carbonyle d'un composé de formule générale IV,

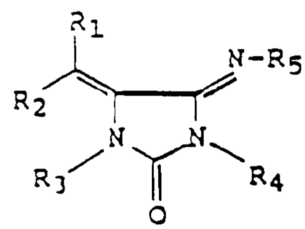

(IV)

dans laquelle $R_1$, $R_2$, $R_3$, $R_4$ et $R_5$ ont les significations mentionnées ci-dessus, de manière connue en soi, en un groupe thiocarbonyle ou imino,

ou bien

c) dans le cas où, dans le composé de formule générale I, le substituant X est l'oxygène, les substituants $R_3$ et $R_4$ ont les significations mentionnées ci-dessus, mais ne peuvent représenter simultanément un atome d'hydrogène ou un groupe phényle, on fait réagir un composé de formule générale V ou VI.

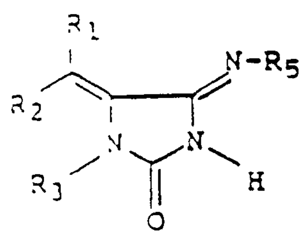

(V)

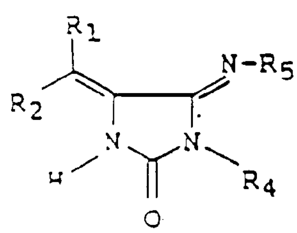

(VI)

dans laquelle $R_1$, $R_2$, $R_3$, $R_4$ et $R_5$ ont les significations mentionnées ci-dessus, de manière connue en soi, avec un composé de formule générale VII,

Y-R6 (VII)

dans laquelle Y a la signification mentionnée ci-dessus et $R_6$ a la même signification que $R_3$ ou $R_4$, excepté la représentation d'un atome d'hydrogène et d'un groupe phényle,

ou bien

d) on fait réagir un composé de formule générale VIII,

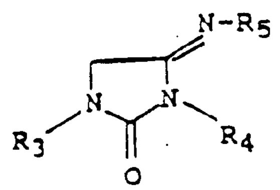

(VIII)

dans laquelle $R_3$, $R_4$ et $R_5$ ont les significations mentionnées ci-dessus, avec un composé de formule générale IX

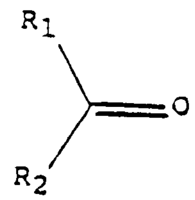

(IX)

dans laquelle $R_1$ et $R_2$ ont les significations mentionnées ci-dessus, à l'aide d'un agent de condensation, ou bien

e) dans le cas où X, dans la formule générale I, représente l'oxygène, on fait réagir un composé de formule générale X,

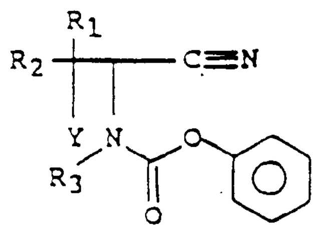

(X)

dans laquelle $R_1$, $R_2$, $R_3$ et Y ont les significations mentionnées ci-dessus, avec une amine primaire de formule générale XI

$R_4$-$NH_2$ (XI)

dans laquelle $R_4$ a la signification mentionnée ci-dessus,

ou bien

f) on transforme des composés de formule générale 1, dans laquelle $R_5$ représente un atome d'hydrogène, éventuellement ultérieurement, par alkylation, en composés de formule générale I, dans laquelle $R_5$ représente un groupe alkyle inférieur,

ou bien

g) dans le cas où $R_1$, $R_2$, $R_3$, $R_4$ et $R_5$, dans la formule générale I, représentent un atome d'hydrogène, on soumet le composé 4-imino- 1,3 -diazabicyclo[3. 1.0]hexan-2-one à une hydrolyse,

ou bien

h) dans le cas où $R_4$ et $R_5$ représentent un atome d'hydrogène et X représente un atome d'oxygène, on fait réagir un composé de formule générale XII

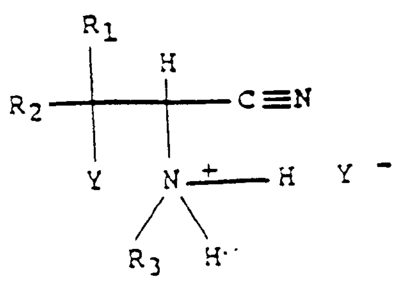

(XII)

dans laquelle $R_1$, $R_2$, $R_3$, et Y ont les significations mentionnées ci-dessus, avec l'isocyanate de chlorosulfonyle,

et ensuite, si on le souhaite, on transforme un composé de formule I en un autre composé de formule I.

4. Procédé de préparation de composés de formule I' selon la revendication 3

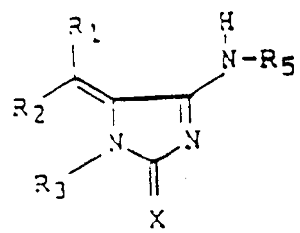

(I')

dans laquelle

$R_1$ et $R_2$ peuvent être identiques ou différents et représentent un atome d'hydrogène ou un groupe alkyle en $C_1$-$C_5$, un groupe alcényle en $C_3$-$C_5$, un groupe phényle ou forment, conjointement avec l'atome de carbone commun, un cycle en $C_3$-$C_7$ saturé ou insaturé,

$R_3$ représente un atome d'hydrogène, un groupe alkyle en $C_1$-$C_{10}$ à chaîne droite ou ramifiée, un groupe alcényle en $C_3$-$C_7$ à chaîne droite ou ramifiée, un groupe cycloalkyle en $C_3$-$C_7$, un groupe cycloalcényle en $C_3$-$C_7$, un groupe phényle, benzyle, picolyle, thényle ou furfuryle,

$R_5$ représente un atome d'hydrogène ou un groupe méthyle, éthyle ou propyle,

et

X représente un atome d'oxygène, un atome de soufre ou un groupe N-H.

5. Médicament contenant au moins 1 des composés selon la revendication 1 ou 2, en plus de véhicules et d'adjuvants usuels.

6. Utilisation de composés selon la revendication 1 ou 2, comme immunosuppresseurs.

7. Utilisation des composés selon la revendication 1 ou 2, pour la préparation de médicaments ayant une activité d'immunosuppresseur.